(19) Europäisches Patentamt
European Patent Office
Office européen des brevets

(11) Publication number: 0 253 498 A2

## (12) EUROPEAN PATENT APPLICATION

(21) Application number: 87305184.1

(22) Date of filing: 11.06.87

(51) Int. Cl.4: C07D 295/02 , C07D 295/06 , C07D 295/04 , C07C 87/457 , A01N 33/04

(30) Priority: 16.07.86 GB 8617377

(43) Date of publication of application: 20.01.88 Bulletin 88/03

(84) Designated Contracting States: AT BE CH DE ES FR GB GR IT LI LU NL SE

(71) Applicant: IMPERIAL CHEMICAL INDUSTRIES PLC
Imperial Chemical House Millbank
London SW1P 3JF(GB)

(72) Inventor: Anthony, Vivienne Margaret
4 The Croft
Maidenhead Berkshire(GB)
Inventor: Worthington, Paul Anthony
4 Oakhurst Road Maidenhead Court Park
Maidenhead Berkshire(GB)

(74) Representative: Alner, Henry Giveen Hamilton et al
Imperial Chemical Industries PLC Legal Department: Patents P.O. Box 6 Bessemer Road
Welwyn Garden City Herts, AL7 1HD(GB)

# (54) Tertiary amine compounds.

(57) Compound of formula:

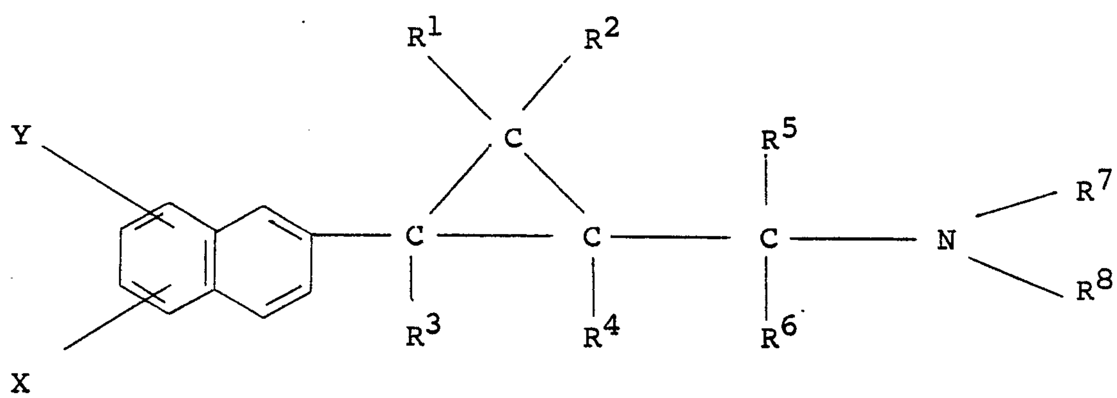

and stereoisomers thereof, wherein $R^1$, $R^2$, $R^3$ and $R^4$ each represent a hydrogen atom, a halogen atom or an alkyl group containing from I to 4 carbon atoms, $R^5$ and $R^6$ each represent a hydrogen atom or an alkyl

EP 0 253 498 A2

Xerox Copy Centre

group containing from 1 to 4 carbon atoms, $R^7$ and $R^8$ each represent an alkyl group containing from 1 to 4 carbon atoms or $R^7$ and $R^8$ together with the adjacent nitrogen atom form a heterocyclic ring which may optionally contain an additional hetero atom, X and Y each represent a hydrogen atom, a halogen atom or an alkyl, cycloalkyl, cycloalkylalkyl, alkenyl, alkynyl, aryl, aralkyl, alkoxy, aryloxy, aralkoxy or haloalkyl group, or a

$$R^9 - \underset{R^{11}}{\overset{R^{10}}{\overset{|}{\underset{|}{Si}}}} -$$

group wherein $R^9$, $R^{10}$ and $R^{11}$ can be alkyl, alkenyl, alkynyl, cycloalkyl, cycloalkylalkyl, or aryl have plant fungicidal activity.

## TERTIARY AMINE COMPOUNDS

This invention relates to tertiary amine compounds containing a cyclopropane ring which are useful as fungicides, to a process for preparing the compounds, to fungicidal compositions containing them, and to methods of using them to combat fungi, especially fungal infections in plants.

According to the invention there are provided compounds having the general formula (I):

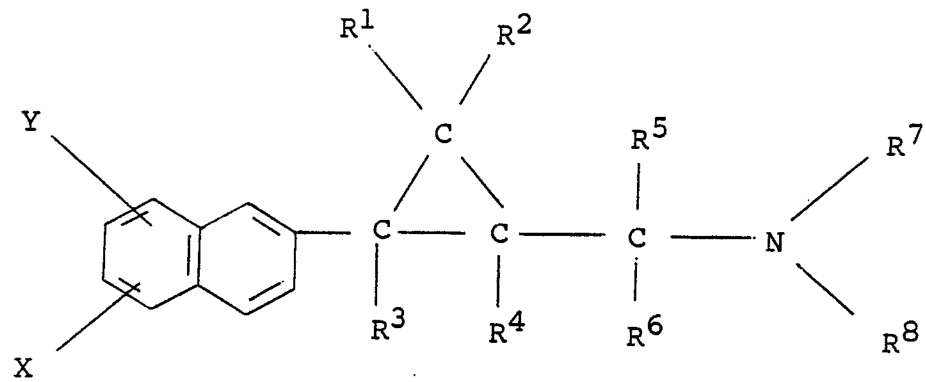

and stereoisomers thereof, wherein $R^1$, $R^2$, $R^3$ and $R^4$ each represent a hydrogen atom, a halogen atom or an alkyl group containing from l to 4 carbon atoms, $R^5$ and $R^6$ each represent a hydrogen atom or an alkyl group containing from l to 4 carbon atoms, $R^7$ and $R^8$ each represent an alkyl group containing from l to 4 carbon atoms or $R^7$ and $R^8$ together with the adjacent nitrogen atom form a heterocyclic ring which may optionally contain an additional hetero atom, X and Y each represent a hydrogen atom, a halogen atom or an alkyl, cycloalkyl, cycloalkylalkyl, alkenyl, alkynyl, aryl, aralkyl, alkoxy, aryloxy aralkoxy or haloalkyl group, or a

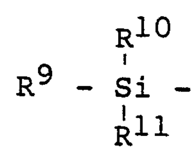

group wherein $R^9$, $R^{10}$ and $R^{11}$ can be alkyl, alkenyl, alkynyl, cycloalkyl, cycloalkylalkyl or aryl. A preferred silyl group is trimethylsilyl, ie. one in which $R^9$, $R^{10}$ and $R^{11}$ are all methyl groups.

The compounds have fungicidal activity.

The compounds of the invention are generally obtained in the form of mixtures of geometric isomers. However, these and other mixtures of optical isomers can be separated into individual isomers by methods in the art and such isomers constitute a part of the present invention.

The invention also includes the acid addition salts of the compounds of formula (I).

When $R^7$ and $R^8$, together with the adjacent N-atom, represent a heterocyclic ring this may be, for example, a piperidine, morpholine, thiomorpholine, pyrrolidine or piperazine ring and any of these rings may bear substitutents such as one or more $C_{1-4}$ alkyl groups or a phenyl group, or a hydroxy$C_{1-4}$alkyl group.

Alkyl groups containing from l to 4 carbon atoms represented by $R^1$ to $R^8$ may be either straight or branched chain groups, for example methyl, ethyl, <u>n</u>-propyl, <u>iso</u> -propyl, <u>n</u>-butyl, <u>sec</u>-butyl, <u>iso</u> -butyl or <u>tert</u>-butyl. Alkyl and alkoxy groups represented by X and Y but also for $R^9$ to $R^{11}$ when alkyl may be either straight or branched chain groups containing from l to 6 carbon atoms; cycloalkyl groups represented by either X or Y and also for $R^9$ to $R^{11}$ may be a $C_3$ to $C_6$ cycloalkyl group, for example a cyclopropyl, cyclobutyl, cyclopentyl or cyclohexyl group.

The halogen atoms which $R^1$ to $R^4$ and X and Y may represent may be fluorine, chlorine or bromine.

Alkenyl and alkynyl groups represented by X and Y and also for $R^9$ to $R^{11}$ may contain from 2 to 6 carbon atoms.

Examples of X and Y when this is aryl, aralkyl, aryloxy or aralkoxy groups are phenyl, benzyl, phenoxy and benzyloxy. These rings may be substituted with halogen (eg. fluorine, chlorine, or bromine), $C_{1-6}$ alkyl e.g. methyl, ethyl, propyl (n-or iso-propyl) and butyl [n -, sec-, iso-, or t-butyl)], $C_{1-6}$ alkoxy (eg. methoxy, ethoxy, propoxy and butoxy) halo-$C_{1-6}$-alkoxy (eg. trifluoromethoxy), halo-$C_{1-6}$alkyl (e.g. trifluoromethyl) nitro, phenyl and phenoxy. The phenyl ring may thus be unsubstituted or substituted with ring substituents as defined above.

Haloalkyl groups represented by X and Y may be for example, chloromethyl, fluoromethyl, chloroethyl, fluoroethyl, trichloromethyl or trifluoromethyl groups.

The acid addition salts of the compounds of the invention may be salts with inorganic or organic acids for example hydrochloric, nitric, sulphuric, acetic, 4-toluenesulphonic or oxalic acid.

Examples of compounds according to the invention are given in Table I.

TABLE I

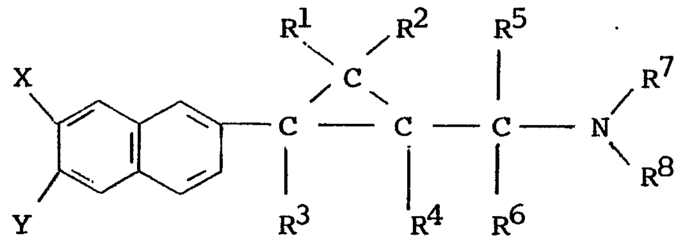

| Compound No. | X | Y | $R^1$ | $R^2$ | $R^3$ | $R^4$ | $R^5$ | $R^6$ | 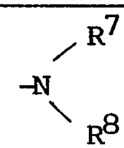 | mp/bp (°C) | Comments |
|---|---|---|---|---|---|---|---|---|---|---|---|
| 1 | H | H | H | H | H | H | H | H | 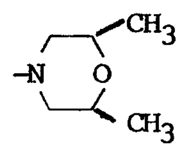 | oil | trans $Ar/CH_2$ cis $CH_3/CH_3$ |
| 2 | H | H | H | H | H | H | H | H | 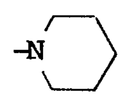 | oil | trans $Ar/CH_2$ |
| 3 | H | t-Bu | H | H | H | H | H | H | 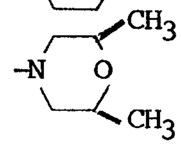 | | trans $Ar/CH_2$ cis $CH_3/CH_3$ |

TABLE I (cont)

| Compound No. | X | Y | $R^1$ | $R^2$ | $R^3$ | $R^4$ | $R^5$ | $R^6$ | 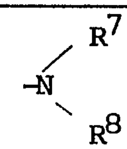 | mp/bp (°C) | Comments |
|---|---|---|---|---|---|---|---|---|---|---|---|
| 4 | t-Bu | H | H | H | H | H | H | H | 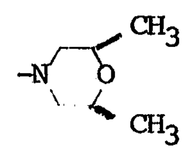 | | trans $Ar/CH_2$ cis $CH_3/CH_3$ |
| 5 | H | H | H | H | H | H | H | H | 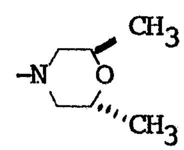 | | trans $Ar/CH_2$ trans $CH_3/CH_3$ |
| 6 | H | H | H | H | H | H | H | H | 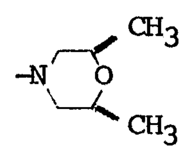 | | cis $Ar/CH_2$ cis $CH_3/CH_3$ |
| 7 | H | H | H | H | H | H | H | H | 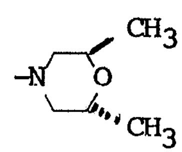 | | cis $Ar/CH_2$ trans $CH_3/CH_3$ |
| 8 | $CH_3$ | H | H | H | H | H | H | H | 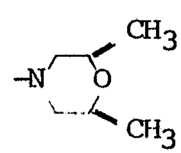 | | trans $Ar/CH_2$ cis $CH_3/CH_3$ |

TABLE I (cont)

| Compound No. | X | Y | $R^1$ | $R^2$ | $R^3$ | $R^4$ | $R^5$ | $R^6$ | 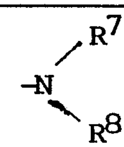 | mp/bp (°C) | *Comments |
|---|---|---|---|---|---|---|---|---|---|---|---|
| 9 | $CH_3$ | H | H | H | H | H | H | H | 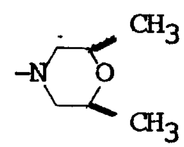 | | cis $Ar/CH_2$<br>cis $CH_3/CH_3$ |
| 10 | H | $CH_3$ | H | H | H | H | H | H | 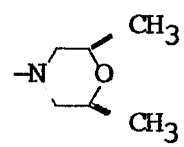 | | trans $Ar/CH_2$<br>cis $CH_3/CH_3$ |
| 11 | H | $CH_3$ | H | H | H | H | H | H | 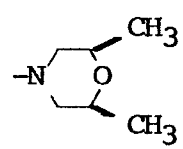 | | cis $Ar/CH_2$<br>cis $CH_3/CH_3$ |
| 12 | t-Bu | H | H | H | H | H | H | H | 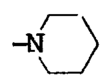 | | trans $Ar/CH_2$ |
| 13 | t-Bu | H | H | H | H | H | H | H | 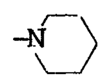 | | cis $Ar/CH_2$ |

TABLE I (cont)

| Compound No. | X | Y | $R^1$ | $R^2$ | $R^3$ | $R^4$ | $R^5$ | $R^6$ | 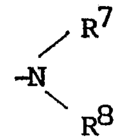 | mp/bp (°C) | Comments |
|---|---|---|---|---|---|---|---|---|---|---|---|
| 14 | H | t-Bu | H | H | H | H | H | H | 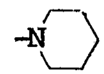 | | trans $Ar/CH_2$ |
| 15 | H | t-Bu | H | H | H | H | H | H | 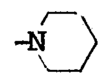 | | cis $Ar/CH_2$ |
| 16 | Cl | H | H | H | H | H | H | H | 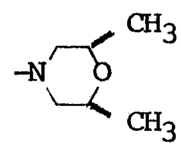 | | trans $Ar/CH_2$<br>cis $CH_3/CH_3$ |
| 17 | Cl | H | H | H | H | H | H | H | 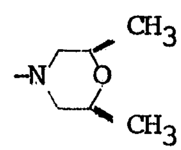 | | cis $Ar/CH_2$<br>cis $CH_3/CH_3$ |
| 18 | H | Cl | H | H | H | H | H | H | 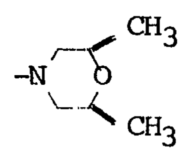 | | trans $Ar/CH_2$<br>cis $CH_3/CH_3$ |

TABLE I (cont)

| Compound No. | X | Y | $R^1$ | $R^2$ | $R^3$ | $R^4$ | $R^5$ | $R^6$ | 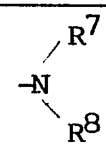 | mp/bp (°C) | Comments |
|---|---|---|---|---|---|---|---|---|---|---|---|
| 19 | H | Cl | H | H | H | H | H | H | 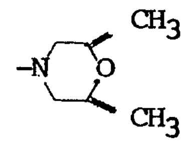 | | cis $Ar/CH_2$ cis $CH_3/CH_3$ |
| 20 | $(CH_3)_3Si$ | H | H | H | H | H | H | H | 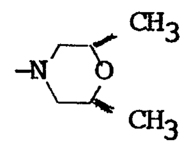 | | trans $Ar/CH_2$ cis $CH_3/CH_3$ |
| 21 | $(CH_3)_3Si$ | H | H | H | H | H | H | H | 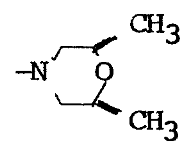 | | cis $Ar/CH_2$ cis $CH_3/CH_3$ |
| 22 | H | $(CH_3)_3Si$ | H | H | H | H | H | H | 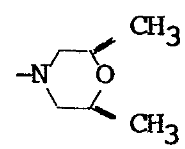 | | trans $Ar/CH_2$ cis $CH_3/CH_3$ |
| 23 | H | $(CH_3)_3Si$ | H | H | H | H | H | H | 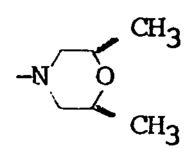 | | cis $Ar/CH_2$ cis $CH_3/CH_3$ |

Compounds of general formula (I) can be prepared by treatment of a compound of general formula (II):

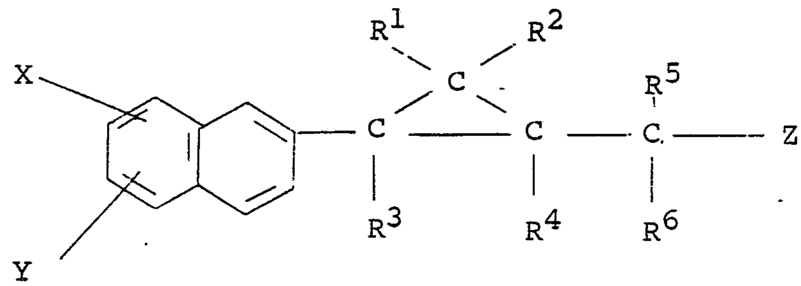

(II)

wherein $R^1$ to $R^6$, X and Y are as defined above and Z is a leaving group such as chlorine, bromine, mesylate or tosylate, with an amine of general formula (III):

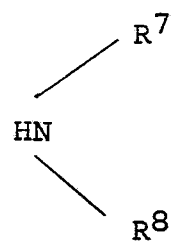

(III)

wherein $R^7$ and $R^8$ are as defined above, in the presence of a convenient solvent such as ethanol or tetrahydrofuran, or, preferably, in the absence of a solvent at a temperature of 20-100°C. The compounds (II) can be prepared by treating an alcohol of general formula (IV):

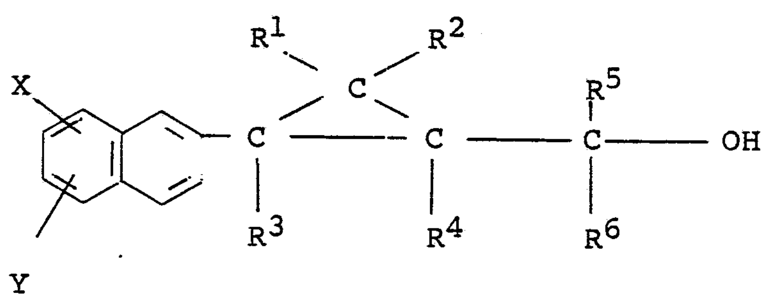

(IV)

wherein $R^1$ to $R^6$, X and Y are as defined above, with the usual halogenating agents (for example phosphorus trichloride, phosphorus pentachloride, or phosphorus oxychloride when Z = chlorine and phosphorus tribromide, phosphorus pentabromide, or phosphorus oxybromide when Z = bromine), or mesyl chloride in pyridine (or triethylamine) when Z = mesylate, or tosyl chloride in pyridine (or triethylamine);when Z = tosylate.

The alcohols (IV), wherein $R^5$ = $R^6$ = hydrogen, can be prepared by treating an ester of general formula (V):

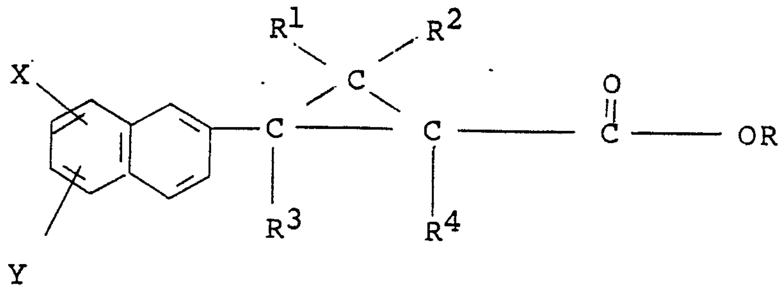

(V)

wherein $R^1$, $R^2$, $R^3$ $R^4$, X and Y are as defined above and R is alkyl with a reducing agent (usually lithium aluminium hydride) in a convenient solvent such as diethyl ether or tetrahydrofuran.

The alcohols (IV), wherein $R^5$ = $R^6$ = alkyl, can be prepared by treating the above ester (V) with an excess of alkyl magnesium halide or an excess of alkyl lithium in a convenient solvent such as diethyl ether or tetrahydrofuran.

The cyclopropane esters (V) can be prepared by reacting the unsaturated esters of general formula (VI):

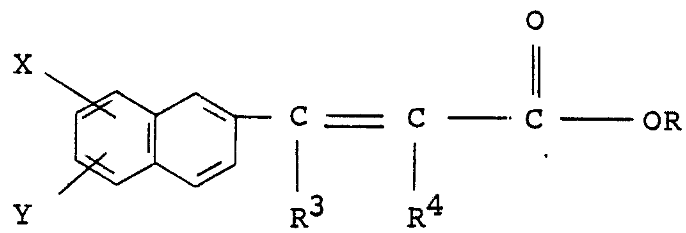

(VI)

wherein R, $R^3$, $R^4$, X and Y are as defined above, with a phosphorane of general formula (VII):

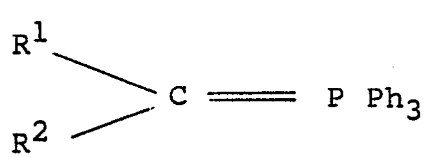

(VII)

wherein $R^1$ and $R^2$ are as defined above except that $R^1$ and $R^2$ cannot be halogen atoms in a convenient solvent such as tetrahydrofuran.

In an alternative process the esters of general formula (V) wherein $R^4$ = hydrogen can be prepared by adding ethyl diazoacetate to the olefin of general formula (VIII):

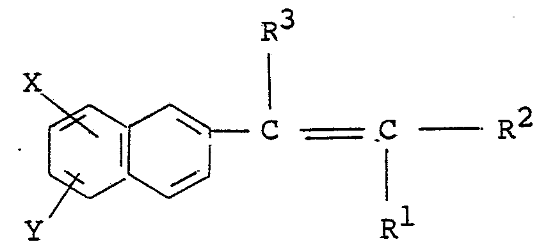

(VIII)

wherein $R^1$, $R^2$, $R^3$, X and Y are as defined above, in a convenient solvent such as chloroform or dichloromethane using an appropriate catalyst such as anhydrous copper sulphate.

The compounds of general formula (I) wherein $R^5$ = $R^6$ = hydrogen can also be prepared by treating an amide of general formula (IX):

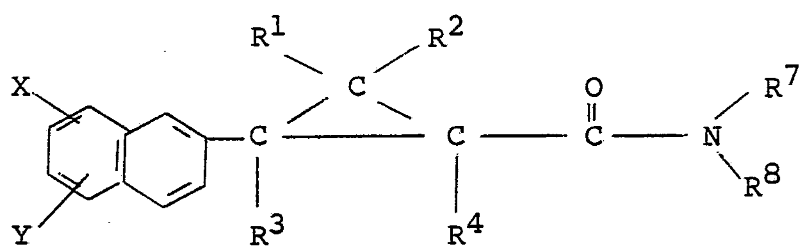

(IX)

wherein $R^1$ to $R^4$, $R^7$ and $R^8$, X and Y are as defined above, with a reducing agent (usually lithium aluminium hydride) in a convenient solvent such a diethyl ether or tetrahydrofuran.

The amides of general formula (IX) can be prepared by reacting an acid chloride of general formula (X):

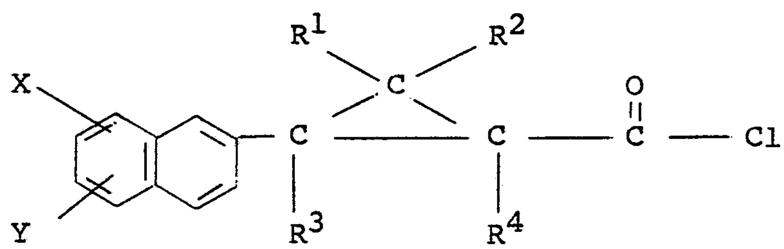

(X)

wherein $R^1$ to $R^4$, X and Y are as defined above, with an amine of general formula (III):

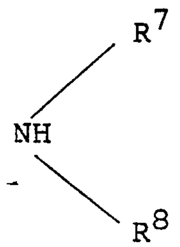

(III)

in a convenient solvent such as diethyl ether or tetrahydrofuran.

The acid chlorides of general formula (X) can be prepared by reacting an acid of general formula (XI):

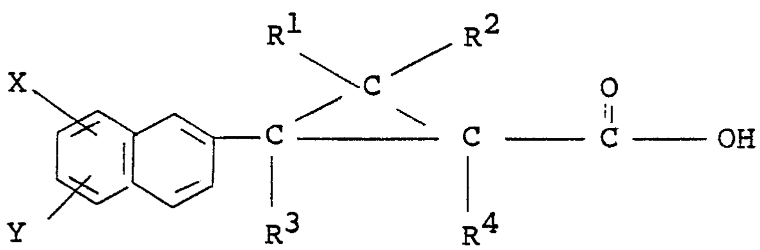

(XI)

wherein $R^1$ to $R^4$, X and Y are as defined above, with the usual chlorinating agents such as thionyl chloride or oxalyl chloride in a convenient solvent such as hexane or dichloromethane.

The acids of general formula (XI) can be prepared by hydrolysing the esters (V) in the normal manner using either sodium or potassium hydroxide in methanol/water solution at 20-60°C.

It is normal for compounds of general formula (IV) when $R^1$ = $R^2$ = halogen to be prepared from the substituted allyl alcohol (XII):

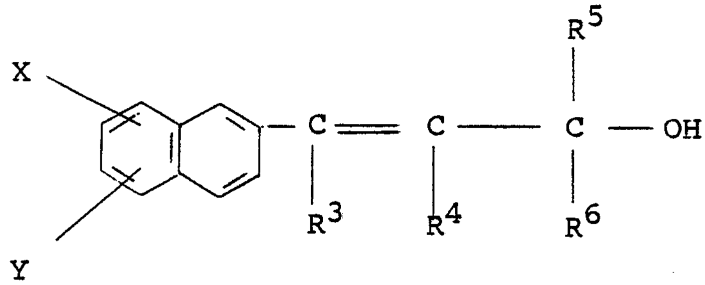

(XII)

wherein $R^3$ to $R^6$, X and Y are as defined above by the addition of a dihalocarbene in a convenient solvent such as chloroform or dichloromethane.

Alcohols of general formula (IV) can be prepared from the allylic alcohols of general formula (XII) using the conditions of the Simmons-Smith reaction.

The salts of compounds of general formula (I) may be prepared from the latter by known methods.

The compounds of formula I and compositions containing them, are variously active against a wide range of fungal diseases, particularly, for example, against Puccinia recondita, Puccinia striiformis and other rusts on wheat, Puccinia hordei, Puccinia striiformis and other rusts on barley, and rusts on other hosts e.g. coffee, pears, apples, vegetables and ornamental plants.

Erysiphe graminis (powdery mildew) on barley and wheat and other powdery mildews on various hosts such as Spaerotheca macularis on hops. Sphaerotheca fuliginea on cucurbits (e.g. cucumber) Podosphaera leucotricha on apples and Uncinula necator on vines.

Cercospora arachidicola and Cercosporidiumpersonata on peanuts and other Cercospora species on for example sugar beet, bananas and soya beans and rice.

Venturia inaequalis (scab) on apples.

Some of the compounds have also shown a broad range of activities against fungi in vitro. They have activity against various post-harvest diseases of fruit (e.g. Botrytis cinerea on grape, Penicillium digitatum and italicum, and Trichoderma viride on oranges and Gloeosporium musarumon bananas).

The compounds can move acropetally in the plant tissue. Moreover, the compounds can be volatile enough to be active in the vapour phase against fungi on the plant.

The compounds may also be useful as industrial (as opposed to agricultural) fungicides, e.g. in the prevention of fungal attack on wood, hides, leather and especially paint films.

This invention, therefore, includes the foregoing uses of the compounds (and compositions containing them) in addition to their principal use as fungicides.

The compounds may be used as such for fungicidal purposes but are more conveniently formulated into compositions for such usage. The invention thus provides a fungicidal composition comprising a compound of general formula (I) as hereinbefore defined; or a salt thereof, and, optionally, a carrier or diluent.

The invention also provides a method of combating fungi, which comprises applying to a plant, to seed of a plant, or to the locus of the plant or seed, a compounds as hereinbefore defined, or a composition containing the same.

The compounds can be applied in a number of ways. For example they can be applied, formulated or unformulated, directly to the foliage of a plant, to seeds or to other medium in which plants are growing or are to be planted. They can be sprayed on, dusted on or applied as a cream or paste formulation, or they can be applied as a vapour or as slow release granules. Application can be to any part of the plant including the foliage, stem, branches or roots, or to soil surrounding the roots, or to the seed before it is planted; or to the soil generally, to paddy water or to hydroponic culture systems. The invention compounds may also be injected into plants or sprayed onto vegetation using electrodynamic spraying techniques or other low volume methods.

The term "plant" as used herein includes seedlings, bushes and trees. Furthermore, the fungicidal methods of the invention includes preventative, protectant, prophylactic and eradicant treatment.

The compounds are preferably used for fungicidal and horticultural purposes in the form of a composition. The type of composition used in any instance will depend upon the particular purpose envisaged.

The compositions may be in the form of dustable powders or granules comprising the active ingredient (invention compound) and a solid diluent or carrier, for example fillers such as kaolin, bentonite, kieselguhr, dolomite, calcium carbonate, talc, powdered magnesia, Fuller's earth, gypsum, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed may include an agent (for example a mineral oil) for assisting the adhesion of the composition to the seed; alternatively the active ingredient can be formulated for seed dressing purposes using an organic solvent (for example N-methylpyrrolidone, propylene glycol or dimethylformamide). The compositions may also be in the form of wettable powders or water dispersible granules comprising wetting or dispersing agents to facilitate their dispersion in liquids. The powders and granules may also contain fillers and suspending agents.

Emulsifiable concentrates or emulsions may be prepared by dissolving the active ingredient in an organic solvent optionally containing a wetting or emulsifying agent and then adding the mixture to water which may also contain a wetting or emulsifying agent. Suitable organic solvents are aromatic solvents such as alkylbenzenes and alkylnaphthalenes, ketones such as isophorone, cyclohexanone and methylcyclohexanone, chlorinated hydrocarbons such as chlorobenzene and trichloroethane, and alcohols such as furfuryl alcohol, butanol and glycol ethers.

Suspension concentrates of largely insoluble solids may be prepared by ball or bead milling with a dispersing agent and including a suspending agent to stop the particles of the solid settling.

Compositions to be used as sprays may be in the form of aerosols wherein the formulation is held in a container under pressure in the presence of a propellant, e.g. fluorotrichloromethane or dichlorodifluoromethane.

The invention compounds can be mixed in the dry state with a pyrotechnic mixture to form a composition suitable for generating in enclosed spaces a smoke containing the compounds.

Alternatively, the compounds may be used in micro-encapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities.

The invention compounds can be used as mixtures with fertilisers (e.g. nitrogen-, potassium-or phoshorus-containing fertilisers). Compositions comprising only granules of fertiliser incorporating, for example coated with, the compound are preferred. Such granules suitably contain up to 25% by weight of the compound. The invention therefore also provides a fertiliser composition comprising a fertiliser and the compound of general formula (I) or a salt thereof.

Wettable powders, emulsifiable concentrates and suspension concentrates will normally contain surfactants e.g. a wetting agent, dispersing agent, emulsifying agent or suspending agent. These agents can be cationic, anionic or non-ionic agents.

Suitable cationic agents are quaternary ammonium compounds, for example cetyltrimethylammonium bromide. Suitable anionic agents are soaps, salts of aliphatic momoesters of sulphuric acid (for example sodium lauryl sulphate), and salts of sulphonated aromatic compounds (for example sodium dodecylbenszenesulphonate, sodium, calcium or ammonium lignosulphonate, butylnaphthalene sulphonate, and a mixture of sodium diisopropyl-and triisopropyl-naphthalene sulphonates).

Suitable non-ionic agents are the condensation products of ethylene oxide with fatty alcohols such as olely or cetyl alcohol, or with alkyl phenols such as octyl-or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and swelling clays such as bentonite or attapulgite.

Compositions for use as aqueous dispersions or emulsions are generally supplied in the form of a concentrate containing a high proportion of the active ingredient, the concentrate being diluted with water before use. These concentrates should preferably be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably l0-85% for example 25-60%, by weight of the active ingredient. After dilution to form aqueous preparations, such preparations may contain varying amounts of the active ingredient depending upon the intended purpose, but an aqueous preparation containing 0.0005% or 0.0l% by weight of active ingredient may be used.

The compositions of this invention may contain other compounds having biological activity, e.g. compounds having a similar or complementary fungicidal activity or plant posses plant growth regulating, herbicidal or insecticidal activity.

A fungicidal compound which may be present in the composition of the invention may be one which is capable of combating ear disease of cereals (e.g. wheat) such as Septoria, Gibberella and Helminthosporium spp., seed and soil-borne diseases and downy and powdery mildews on grapes and powdery mildew and scab on apple etc. By including another fungicide, the composition can have a broader spectrum of activity than the compound of general formula (I) alone. Further to the other fungicide can have a synergistic effect on the fungicidal activity of the compound of general formula (I). Examples of fungicidal compounds which may be included in the composition of the invention are carbendazim, benomyl, thiophanate-methyl, thiabendazole, fuberidazole, etridiazole, dichlorofluanid, cymoxanil, oxadixyl, ofurace, metalaxyl, fualaxyl, benalaxyl, fosetyl aluminium, fenarimol, iprodione, procymidone, vinclozolin, penconazole, myclobutanil, RO151297, pyrazophos, ethirimol, ditalimfos, tridemorph, triforine, nuarimol, triazbutyl, guazatine, triacetate salt of l,l-iminodi (octamethylene)diguanidine, propiconazole, prochloraz, flutriafol, hexaconazole i.e. the chemical l-(l,2,4-triazol-l-yl)-2-(2,4-dichlorophenyl)hexan-2-ol, DPX H6573 (l-((bis-4-fluorophenyl)methylsilyl)methyl)-lH-l,2,4-triazole, triadimefon, triadimenol, diclobutrazol, fenpropimorph, fenpropidin, pyrifenox, ( 2RS,3RS)-2-(4-chlorophenyl)-3-cyclopropyl-l-(lH-l,2,4-triazol-l-yl)-butan-2-ol, (RS)-l-(4-chlorophenyl)-4,4-dimethyl-3-(lH-l,2,4-triazol-l-ylmethyl)pentan-3-ol, 4-chloro-N-(cyano(ethoxy)methyl)-benzamide, chlorozolinate, diniconazol, imazalil, fenfuram, carboxin, oxycarboxin, methfuroxam, dodemorph, BAS 454, blasticidin S, kasugamycin, edifenphos, kitazin -P, cycloheximide, phthalide, probenzazole, isoprothiolane, tricyclazole, pyroquilon, chlorbenzthiazone, neoasozin, polyoxin D, validamycin A, mepronil, flutolanil, pencycuron, diclomezine, phenazin oxide, nickel dimethyldithiocarbamate, techlofthalam, bitertanol, bupirimate, etaconazole, streptomycin, cypofuram, bioloxiazol, quinomethionate, dimethirimol, l-(2-cyano-2-methoxyimino-acetyl)-3-ethyl urea, fenapanil, tolclofos-methyl, pyroxyfur, polyram, maneb, mancozeb, captafol, chlorothalonil, anilazine, thiram, captan, folpet, zineb, propineb, sulphur, dinocap, binapactryl, nitrothalisopropyl, dodine, dithianon, fentin hydroxide, fentin acetate, tecnazene, quintozene, dichloran, copper containing compounds such as copper oxychloride, copper sulphate and Bordeaux mixture, and organomercury compounds.

The compounds of general formula (I) can be mixed with soil, peat or other rooting media for the protection of plants against seed-borne or foliar fungal diseases.

Suitable insecticides which may be incorporated in the compositon of the invention include pirimicarb, dimethoate, demeton-s-methyl, formothion, carbaryl, isoprocarb, XMC, BPMC, carbofuran, carbosulfan, diazinon, fenthion, fenitrothion, phenthoate, chlopyrifos, isoxathion, propaphos, momocrotphas, buprofezin, ethroproxyfen and cycloprothrin.

Plant growth regulating compounds are compounds which control weeds or seedhead formation, or selectively control the growth of less desirable plants (e.g. grasses).

Examples of suitable plant growth regulating compounds for use with the invention compounds are the gibberellins (e.g. $GA_3$, $GA_4$ or $GA_7$), the auxins (e.g. indoleacetic acid, indolebutyric acid, naphthoxyacetic acid or naphthylacetic acid), the cytokinins (e.g. kinetin, diphenylurea, benzimidazole, benzyladenine or benzylaminopurine), phenoxyacetic acids (e.g. 2,4-D or MCPA), substituted benzoic acids (e.g. triiodobenzoic acid), morphactins (e.g. chlorofluoroecol), maleic hydrazide, glyphoste, glyphosine, long chain fatty alcohols and acids, dikegulac, paclobutrazol, flurprimidiol, fluoridimid, mefluidide, substituted quaternary ammonium and phosphonium compounds (e.g. choromequat chlorphonium or mepiquatchloride), ethephon, carbetamide, methyl-3,6-dichloroanisate, daminozide, asulam, abscisic acid, isopyrimil, l-(4-chlorophenyl)-4,6-dimethyl-2-oxo-l,2-dihydropyridine-3-carboxylic acid, hydroxybenzonitriles (e.g. bromoxynil), difenzoquat, benzoylprop-ethyl 3,6-dichloropicolinic acid, fenpentezol, inabenfide, triapenthenol and tecnazene.

The following Examples illustrate the invention

EXAMPLE I

This Example illustrates the preparation of 4-[2-(naphth-2-yl)-trans-cyclopropylmethyl]-2,6-cis -dimethylmorpholine (Compound No. I in Table I). Triethylphosphonoacetate (28.7 g, 0.l28 mol) in sodium dried diethyl ether (200 ml) was added dropwise at -5°C to a suspension of sodium hydride (6.2 g 0.l29 mol) in sodium dried ether (220 ml). The mixture was stirred at -5°C for l½ hours, then warmed to 0°C and 2-naphthaldehyde (20 g, 0.l28 mol) dissolved in sodium dried ether (200 ml) added dropwise. After the addition the mixture was stirred at room temperature for 3 hours, poured into water and extracted with diethyl ether. The ethereal extracts were washed with water, dried over anhydrous sodium suphate and the ether removed to give ethyl trans -3-(naphth-2-yl)prop-2-enoate (28.9 g, l00%) as a colourless oil.

Ethyl trans-3-(naphth-2-yl)prop-2-enoate (l5 g, 0.066 mol) in sodium dried toluene (65 ml) was stirred at 0°C under a nitrogen atmosphere and diisobutylaluminium hydride (98 ml, 0.l47 mol - l.5 m solution in toluene) was added dropwise. After complete addition the mixture was stirred at 0°C for ½ hour and methanol (30 ml) was added dropwise. After a further ½ hour water and 2N HCl was added to dissolve the precipitate and the solution extracted with diethyl ether. The ethereal extracts were washed with water, dried over anhydrous sodium sulphate. Removal of the solvent gave trans-3-(naphth-2-yl)prop-2-en-l-ol as a white solid (ll.62 g, 96%).

A mixture of trans-3-(naphth-2-yl)prop-2-en-l-ol (ll.62 g, 0.063 mol), zinc dust (l6.38 g, 0.25 mol), copper (I) chloride (2.52 g, 0.025 mol) and dibromomethane (22.05g, 0.l27 mol), in sodium dried ether (40 ml) under nitrogen were sonocated at 45 - 50°C for 2 hours. The mixture was diluted with ether and neutralised with saturated ammonium chloride. The ether layer was washed with water and dried over anhydrous sodium sulphate. Removal of the solvent gave an oil which was purified by column chromatography (silica eluted with ethyl acetate/petroleum ether l:l) to give trans-2-(naphth-2-yl)cyclopropanemethanol (6.8l g, 55%) as a yellow solid.

A mixture of trans-2-(naphth-2-yl)cyclopropanemethanol (6.7 g, 0.034 mol), triphenylphosphine (l7.87 g, 0.068 mol), l,2-dibromotetrachloroethane (22.22g, 0.068 mol), and triethylamine (l3.9 g, 0.l37 mol) in l,2-dichloroethane (l20 ml) were stirred at room temperature for 3 hours. After removal of the solvent at the pump the residue was purified by column chromatography (silica eluted with ethyl acetate/petroleum ether l:l) to give trans-2-(naphth-2-yl)cyclopropanemethyl bromide (8.28 g, 93%) as a yellow oil.

A mixture of trans-2-(naphth-2-yl)cyclopropanemethyl bromide (5 g, 0.0l9 mol) and cis -2,6-dimethylmorpholine (5ml, 0.04 mol) in dry tetrahydrofuran (l00 ml) was refluxed for 2 hours. The mixture was then poured into water, extracted with ether and dried over anhydrous sodium sulphate. Removal of the solvent gave an oil which was purified by column chromatography (silica eluted with ethyl acetate) to give the title compound (0.93 g, l7%).

EXAMPLE 2

This Example illustrates the preparation of l-[2-(naphth-2-yl)-trans-cyclopropylmethyl]-piperidine (Compound No. 2 in Table I).

A mixture of trans-2-(naphth-2-yl)cyclopropanemethyl bromide (3g, 0.0ll5 mol) and piperidine (6 ml, 0.06 mol) in dry tetrahydrofuran (70 ml) was refluxed for 2 hours. The mixture was then poured into water, extracted with ethyl acetate and dried over anhydrous sodium sulphate. Removal of the solvent gave an oil which was purified by column chromatography (silica eluted with ethyl acetate/methanol 9:l) to give the title compound (0.63g, 2l%).

EXAMPLE 3

An emulsifiable concentrate was made up by mixing the ingredients, and stirring the mixture until all the constituents were dissolved.

Compound of Example I l0%
Ethylene dichloride 40%
Calcium dodecylbenzenesulphate 5%
"Lubrol" L l0%
"Aromasol" H 35%

EXAMPLE 4

A composition in the form of grains readily dispersible in a liquid, e.g. water, was prepared by grinding together the first three ingredients in the presence of added water and then mixing in the sodium acetate. The resultant mixture was dried and passed through a British Standard mesh seive, size 44-l00, to obtain the desired size of grains.

Compound of Example I 50%
"Dispersol" T 25%
"Lubrol" APN5 l.5%
Sodium acetate 23.5%

EXAMPLE 5

The ingredients were all ground together to produce a powder formulation readily dispersible in liquids.
Compound of Example I 45%
"Dispersol" T 5%
"Lissapol" NX 0.5%
"Cellofas" B600 2%
Sodium acetate 47.5%

EXAMPLE 6

The active ingredient was dissolved in a solvent and the resultant liquid was sprayed on to the granules of China clay. The solvent was then allowed to evaporate to produce a granular composition.
Compound of Example I 5%
China clay granules 95%

EXAMPLE 7

A composition suitable for use as a seed dressing was prepared by mixing the three ingredients.
Compound of Example I 50%
Mineral oil 2%
China clay 48%

EXAMPLE 8

A dusting powder was prepared by mixing the active ingredient with talc.
Compound of Example I 5%
Talc 95%

EXAMPLE 9

A Col formulation was prepared by ball-milling the constituents set out below and then forming an aqueous suspension of the ground mixture with water.
Compound of Example I 40%
"Dispersol" T I0%
"Lubrol" APN5 I%
Water

EXAMPLE I0

A dispersible powder formulation was made by mixing together the ingredients set out below and then grinding the mixture until all were thoroughly mixed.

Compound of Example I 25%
"Aerosol" OT/B 2%
"Dispersol" A.C. 5%
China clay 28%
Silica 40%

EXAMPLE II

This Example illustrates the preparation of a dispersible powder formulation. The ingredients were mixed and the mixture then ground in a comminution mill.

Compound of Example I 25%
"Perminal" BX I%
"Dispersol" T 5%
Polyvinylpyrrolidone I0%
Silica 25%
China clay 34%

EXAMPLE I2

The ingredients set out below were formulated into a dispersible powder by mixing then grinding the ingredients.

Compound of Example I 25%
"Aerosol" OT/B 2%
"Dispersol" A 5%
China clay 68%

In Examples 3 to I2 the proportions of the ingredients given are by weight.

The compounds set out in Table I and numbered 2 to 23 can be similarly formulated as specifically described in Examples 3 to I2.

There now follows an explanation of the compositions or substances represented by the various Trade Marks mentioned above. LUBROL L :
a condensate of nonyl phenol (I mole) with ethylene oxide (I3 moles)

AROMASOL H :
a solvent mixture of alkylbenzens

DISPERSOL T & AC :
a mixture of sodium sulphate and a condensate of formaldehyde with sodium naphthalene sulphonate

LUBROL APN5 :
a condensate of nonyl phenol (I mole) with naphthalene oxide (5.5 moles)

CELLOFAS B600 :
a sodium carboxymethyl cellulose thickener

LISSAPOL NX :
a condensate of nonyl phenol (I mole) with ethylene oxide (8 moles)

AEROSOL OT/B :
dioctyl sodium sulphosuccinate

PERMINAL BX :
a sodium alkyl naphthalene sulphonate

EXAMPLE I3

The compounds were tested against a variety of foliar fungal diseases of plants. The technique employed was as follows.

The plants were grown in John Innes Potting Compost (No I or 2) in 4 cm diameter minipots. The test compounds were formulated either by bead milling with aqueous Dispersol T or as a solution in acetone or acetone/ethanol which was diluted to the required concentration immediately before use. For the foliage diseases, the formulations (I00 ppm active ingredient) were sprayed on to the foliage and applied to the roots of the plants in the soil. The sprays were applied to maximum retention and the root drenches to a final concentration equivalent to approximately 40 ppm a.i./dry soil. Tween 20, to give a final concentration of 0.05%, was added when the sprays were applied to cereals.

For most of the tests the compound was applied to the soil (roots) and to the foliage (by spraying) one or two days before the plant was inoculated with the disease. An exception was the test on Erysiphe graminis in which the plants were inoculated 24 hours before chemical treatment. Foliar pathogens were applied by spray as spore suspensions onto the leaves of test plants. After inoculation, the plants were put into an appropriate environment to allow infection to proceed and then incubated until the disease was ready for assessment. The period between inoculation and assessment varied from four to fourteen days according to the disease and environment.

The disease control was recorded by the following grading :

4 = no disease
3 = trace - 5% of disease on untreated plants
2 = 6-25% of disease on untreated plants
I = 26-59% of disease on untreated plants
0 = 60-I00% of disease on untreated plants

The results are shown in Table II.

TABLE II

TABLE II

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 1 | 3 | 4 | 3 | 4 |
| 2 | 3 | 0 | 1 | 0 |

**Claims**

I. A compound having the formula (I):

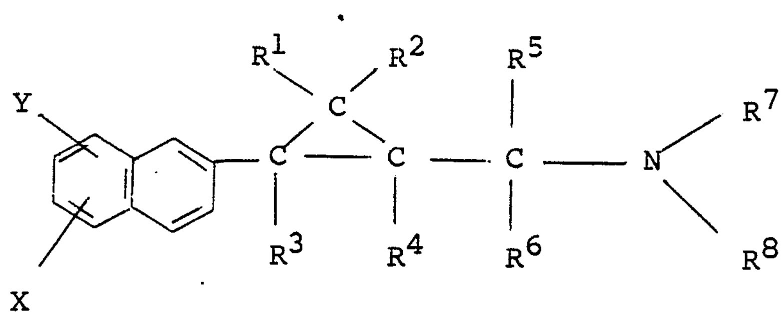

and stereoisomers thereof, wherein $R^1$, $R^2$, $R^3$ and $R^4$ each represent a hydrogen atom, a halogen atom or an alkyl group containing from I to 4 carbon atoms, $R^5$ and $R^6$ each represent a hydrogen atom or an alkyl group containing from I to 4 carbon atoms, $R^7$ and $R^8$ each represent an alkyl group containing from I to 4 carbon atoms or $R^7$ and $R^8$ together with the adjacent nitrogen atom form a heterocyclic ring which may optionally contain an additional hetero atom, X and Y each represent a hydrogen atom, a halogen atom or an alkyl, cycloalkyl, cycloalkylalkyl, alkenyl, alkynyl, aryl, aralkyl, alkoxy, aryloxy, aralkoxy or haloalkyl group, or a

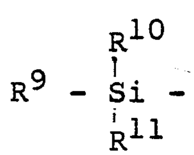

group wherein $R^9$, $R^{10}$ and $R^{11}$ can be alkyl, alkenyl, alkynyl, cycloalkyl, cycloalkylalkyl, or aryl.

2. A compound having the formula (I):

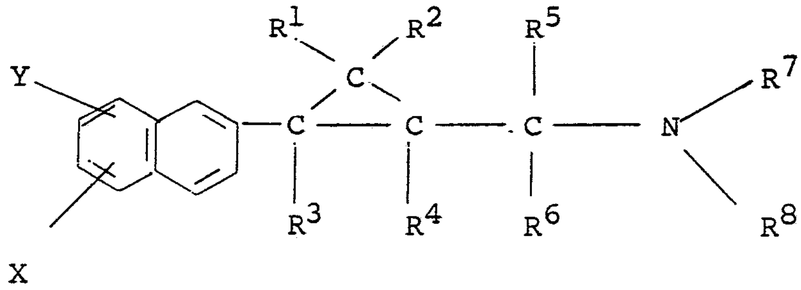

and stereoisomers thereof, wherein $R^1$, $R^2$, $R^3$ and $R^4$ each represent a hydrogen atom, a halogen atom or an alkyl group containing from l to 4 carbon atoms, $R^5$ and $R^6$ each represent a hydrogen atom or an alkyl group containing from l to 4 carbon atoms, $R^7$ and $R^8$ each represent an alkyl group containing from l to 4 carbon atoms or $R^7$ and $R^8$ together with the adjacent nitrogen atom form a piperidine, morpholine, thiomorpholine, pyrrolidine or piperazine ring which may optionally be substituted with one or more $C_{1-4}$ alkyl, hydroxy $C_{1-4}$ alkyl or phenyl groups, X and Y each represent a hydrogen atom, a halogen atom or a $C_{1-6}$ alkyl, $C_{3-6}$ cycloalkyl, $C_{4-7}$cycloalkylalkyl $C_{2-6}$ alkenyl, $C_{2-6}$ alkynyl, phenyl, phenoxy, benzyl, benzyloxy, $C_{1-6}$ alkoxy group, any of which may be substituted with halogen and those with rings with halogen, $C_{1-6}$ alkyl, halo $C_{1-6}$ alkyl, $C_{1-6}$alkoxy, halo $C_{1-6}$ alkoxy, nitro, phenyl or phenoxy, or a

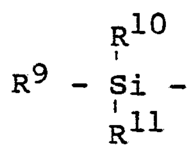

group wherein $R^9$, $R^{10}$ and $R^{11}$ can be $C_{1-6}$ alkyl, $C_{2-6}$ alkenyl, $C_{2-6}$ alkynyl, $C_{3-6}$cycloalkyl $C_{4-7}$ cycloalkylalkyl or phenyl.

3. A compound having the general formula (I):

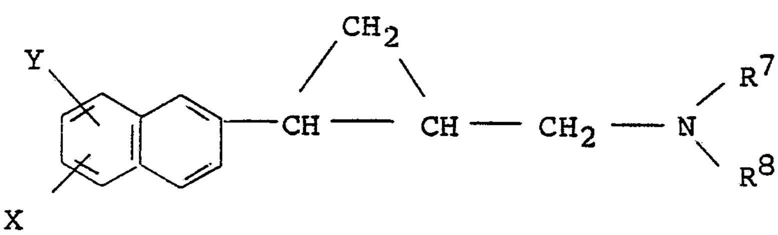

and stereoisomers thereof, wherein $R^7$ and $R^8$ each represent an alkyl group containing from l to 4 carbon atoms or $R^7$ and $R^8$ together with the adjacent nitrogen atom form a morpholine or piperidine ring which may optionally be substituted by a $C_{1-4}$ alkyl group, X and Y each represent a hydrogen atom, a halogen atom or an alkyl group having from l to 4 carbon atoms, or a trimethylsilyl group.

4. 4-[2-(naphth-2-yl)-trans-cyclopropylmethyl]-2,6-cis -dimethylmorpholine.

5. l-[2-(naphth-2-yl)-trans -cyclopropylmethyl]-piperidine.

6. A process for preparing compounds as claimed in any of claims l to 5 which comprises the treatment of a compound of general formula (II)

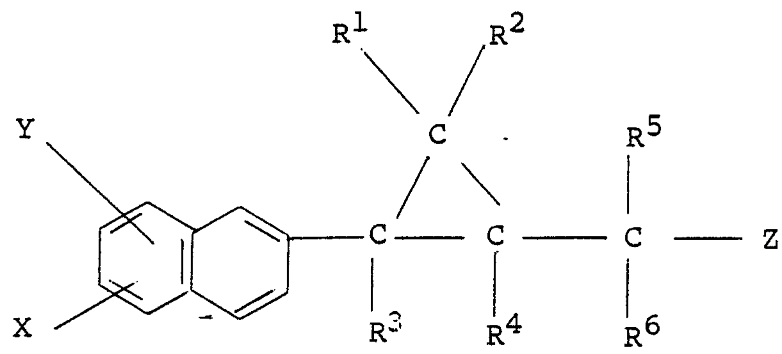

wherein $R^1$ to $R^6$, X and Y are as defined above and Z is a leaving group such as chlorine, bromine, mesylate or tosylate, with an amine of general formula (III):

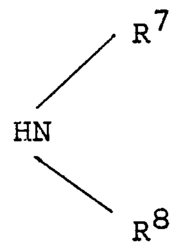

(III)

wherein $R^7$ and $R^8$ are as defined above, in the presence of a convenient solvent such as ethanol or tetrahydrofuran, or, preferably, in the absence of a solvent at a temperature of 20-100°C.

7. A fungicidal composition comprising, as an active ingredient, a compound as claimed in any of claims 1 to 5.

8. A process for combating fungi which comprises applying to a plant, or to the locus of the plant or seed, a compound as claimed in any of claims 1 to 5 or a composition as claimed in claim 7.